# EUROPEAN PATENT SPECIFICATION

(11) **EP 4 049 653 B1**
(45) Date of publication and mention of the grant of the patent: **26.11.2025**
(21) Application number: 20878289.6
(22) Date of filing: 22.10.2020
(51) Int. Cl.: A61K 9/06, A61K 47/36, A61K 9/00, A61F 9/00, C08J 3/075, C08J 3/24, C08B 37/08, C08L 5/08, C08K 5/00

(54) **INJECTABLE INTRAOCULAR MICROGEL AS DRUG DELIVERY SYSTEM, AND HYDROGEL COMPRISING SAME**
INJIZIERBARES INTRAOKULARMIKROGEL ALS WIRKSTOFFFREISETZUNGSSYSTEM UND HYDROGEL DAMIT
MICROGEL INTRAOCULAIRE INJECTABLE SERVANT DE SYSTÈME D'ADMINISTRATION DE MÉDICAMENT, ET HYDROGEL LE COMPRENANT

(30) Priority: 24.10.2019 KR 20190132626
(43) Date of publication of application: 31.08.2022
(73) Proprietor: Seoul National University Hospital, Seoul 03080 (KR); Ajou University Industry-Academic Cooperation Foundation, Gyeonggi-do 16499 (KR)
(72) Inventor: WOO, Se Joon, Seongnam-si Gyeonggi-do 13620 (KR); PARK, Ki Dong, Seoul 06624 (KR); LEE, Si Min, Seoul 03967 (KR); SON, Joo Young, Suwon-si Gyeonggi-do 16503 (KR)
(74) Representative: Petraz, Gilberto Luigi
(86) International application number: PCT/KR2020/014488
(87) International publication number: WO 2021/080345

(56) References cited:
- WO-A1-2012/171335
- WO-A1-2012/171335
- WO-A1-2019/057035
- KR-A- 20110 002 741
- KR-A- 20110 002 741
- KR-A- 20140 037 757
- US-A1- 2010 074 956
- YU Y ET AL: "Inectable chemically crosslinked hydrogel for the controlled release of bevacizumab in vitreous: a 6-month in vivo study", TRANSLATIONAL VISION SCIENCE & TECHNOLOGY,, vol. 4, no. 2, 1 March 2015 (2015-03-01), XP002791841
- EHSANIPOUR ARSHIA ET AL: "Injectable, Hyaluronic Acid-Based Scaffolds with Macroporous Architecture for Gene Delivery", CELLULAR AND MOLECULAR BIOENGINEERING, SPRINGER INTERNATIONAL PUBLISHING, CHAM, vol. 12, no. 5, 4 September 2019 (2019-09-04), pages 399 - 413, XP036898388, ISSN: 1865-5025, [retrieved on 20190904], DOI: 10.1007/S12195-019-00593-0
- ECHALIER C�CILE ET AL: "Chemical cross-linking methods for cell encapsulation in hydrogels", MATERIALS TODAY COMMUNICATIONS, vol. 20, 1 September 2019 (2019-09-01), GB, pages 100536, XP093083862, ISSN: 2352-4928, DOI: 10.1016/j.mtcomm.2019.05.012

## Description

### Technical Field

The present invention relates to a drug delivery system and, more particularly, to an injectable intraocular hydrogel including a microgel, and a method of preparing the hydrogel.

### Background Art

Hydrogels are jelly-type materials having water as a dispersion medium or water as a basic component. Since hydrogels can easily absorb water due to their high hydrophilicity and can easily change their strength and shape, the hydrogels are used as a support for tissue engineering or used for drug delivery, etc. Hydrogels swell by absorbing a large amount of water in an aqueous solution or in an aqueous environment due to the hydrophilicity of the constituent materials thereof but do not dissolve due to the cross-linked structure thereof. Therefore, hydrogels may have various shapes and properties depending on the constituent components and preparing methods thereof and typically contain a large amount of water, so they are positioned as intermediate materials between liquid and solid in terms of characteristics.

On the other hand, in the case of an anti-VEGF agent used for macular degeneration and diabetic macular degeneration among ophthalmic diseases, it is known that the intraocular half-life is short and thus the retention is short. Therefore, in order for the anti-VEGF agent to exhibit the maximum effect, the anti-VEGF agent is required to be frequently injected into the eyes. This not only causes a lot of inconvenience to patients but also imposes a heavy economic burden on patients.

Therefore, after a long period of research and efforts, the inventors have completed the present invention as an injectable intraocular drug delivery system for a protein therapeutic agent for the treatment of ophthalmic diseases.

### Disclosure

### Technical Problem

Disclosed hereion is an injectable intraocular microgel as a drug delivery system that is safe for a human body and that can release a drug in the eye for a long period of time and to provide a preparing method of the microgel.

An objective of the present invention is to provide an injectable intraocular hydrogel including such microgel and to provide a preparing method of the hydrogel.

### Technical Solution

To accomplish the above objectives, a method of preparing an injectable intraocular microgel includes: generating a hyaluronic acid microgel by causing a hyaluronic acid copolymer to undergo a cross-linking reaction through a w/o emulsion method; and loading a drug on the hyaluronic acid microgel.

The generating of the hyaluronic acid microgel may include: synthesizing a hyaluronic acid vinylsulfone (HA-VS) copolymer by causing divinylsulfone (DVS) and hyaluronic acid (HA) to undergo a bonding reaction; and causing the synthesized hyaluronic acid vinylsulfone (HA-VS) copolymer and dithiothreitol (DTT) to undergo a thiol-ene cross-linking reaction.

The generating of the hyaluronic acid microgel may be NHS-NH₂ cross-linking of hyaluronic acid-(N-hydroxy succinimide) (HA-NHS) and hyaluronic acid-amine (HA-NH₂).

The generating of the hyaluronic acid microgel may include: adding horseradish peroxidase (HRP) and hydrogen peroxide (H₂O₂) to hyaluronic acid-tyramine (HA-TA) and causing the mixture to undergo an enzymatic cross-linking reaction.

The generating of the hyaluronic acid microgel may be thiol-ene cross-linking of hyaluronic acid vinylsulfone (HA-VS) and hyaluronic acid thiol (HA-SH).

To accomplish the objectives, an injectable intraocular microgel includes: a hyaluronic acid microgel composed of a hyaluronic acid copolymer cross-linked by a cross-linking functional group represented by at least one selected from the group consisting of Chemical Formulas 1-1 to 1-6 below; and a drug loaded on the hyaluronic acid microgel.

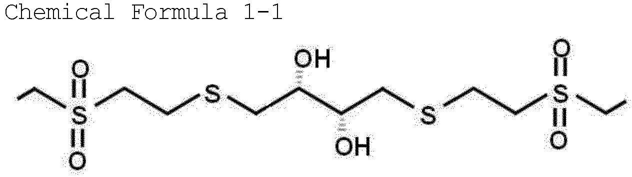

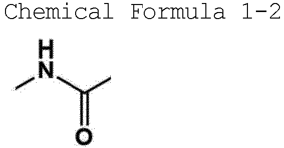

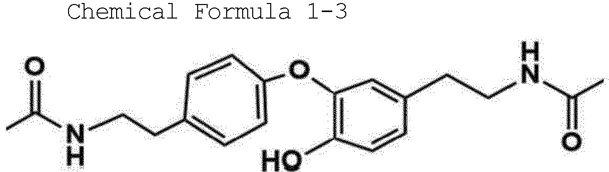

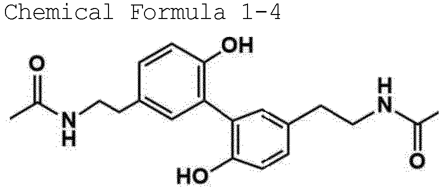

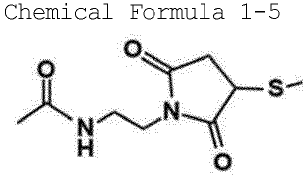

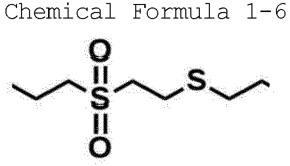

The hyaluronic acid copolymer may be at least one selected from the group consisting of hyaluronic acid vinylsulfone (HA-VS), hyaluronic acid- (N-hydroxy succinimide) (HA-NHS), hyaluronic acid-tyramine (HA-TA), and hyaluronic acid-maleimide (HA-Mal).

The drug may be one or more selected from an injectable intraocular protein therapeutic agent, a chemical drug, a small molecule therapeutic, a gene, and a cell.

The injectable intraocular microgels have an average size of 30 to 160 µm.

According to an embodiment of the present invention to accomplish the objectives, an injectable intraocular hydrogel includes the injectable intraocular microgels dispersed in the hydrogel.

The hydrogel may be cross-linked by a cross-linking functional group represented by at least one selected from the group consisting of Chemical Formulas 2-1 to 2-3 below.

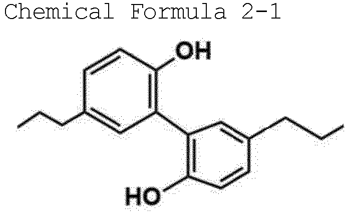

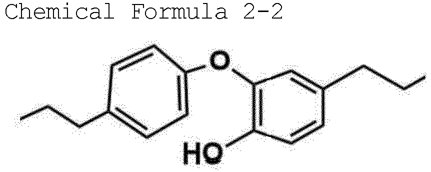

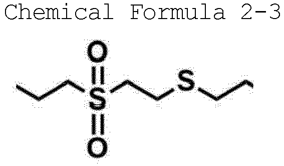

According to a further embodiment of the present invention to accomplish the above objectives, a method of preparing an injectable intraocular hydrogel includes: preparing an injectable intraocular microgel through the method described above; and dispersing the prepared injectable intraocular microgels in the hydrogel.

The injectable intraocular hydrogel preparation method may include: preparing a GPT hydrogel by adding horseradish peroxidase (HRP) and hydrogen peroxide to a gelatin-polyethylene glycol-tyramine (GPT) copolymer so that phenol derivatives in the GPT copolymer cross-link with each other; and mixing the injectable intraocular microgel in the GPT hydrogel.

The injectable intraocular hydrogel preparation method may include: preparing a GH hydrogel by adding horseradish peroxidase (HRP) and hydrogen peroxide to a gelatin-hydroxyphenylacetic acid (GH) copolymer so that phenol derivatives in the GH copolymer cross-link with each other; and mixing the eye-injecting microgel in the GH hydrogel.

The injectable intraocular hydrogel preparation method may include: mixing hyaluronic acid thiol (HA-SH) and hyaluronic acid vinylsulfone (HA-VS) so that a vinyl group of HA-VS and a thiol group of HA-SH cross-link with each other through a thiol-ene reaction, thereby obtaining a HA-VS/HA-SH hydrogel; and mixing the injectable intraocular microgel in the HA-VS/HA-SH hydrogel.

Details are disclosed in the following detailed description and the accompanying drawings.

### Advantageous Effects

The eye-injecting microgel of the present invention has a structure in which a drug is loaded on a hyaluronic acid microgel. Therefore, the injectable intraocular microgel has high in vivo stability and the loaded drug is released in a sustained-release form for more than 30 days. This minimizes the inconvenience of a patient being injected.

In addition, the injectable intraocular hydrogel of the present invention has a structure in which drug-loaded hyaluronic acid microgels are distributed inside the GPT hydrogel. It is also confirmed that injectable intraocular hydrogel has high in vivo stability and the loaded drug is slowly released over a long period of time. Furthermore, the injectable intraocular hydrogel can control the gelation time or mechanical strength by controlling the concentration of horseradish peroxidase or hydrogen peroxide.

### Description of Drawings

FIG. 1 is a schematic diagram illustrating a process in which hyaluronic acid (HA) and divninylsulfone (DVS) are synthesized;
FIG. 2 is a diagram illustrating the formation of a hyaluronic acid microgel using HA-VS and DTT;
FIG. 3 is a ¹H NMR spectrum of a hyaluronic acid vinylsulfone (HA-VS) copolymer;
FIG. 4 is a CLSM image of a hyaluronic acid microgel;
FIG. 5 is a graph illustrating the measurements of the drug release behavior of a drug-loaded hyaluronic acid microgel;
FIG. 6 illustrates the measurement results of the change in the concentration of ranibizumab in vitreous humor of the experimental group and the control group;
FIG. 7 illustrates the measurements of the change in the concentration of ranibizumab in vitreous humor to retina of the experimental group and the control group;
FIG. 8 illustrates the measurements of the change in the concentration of ranibizumab in anterior chamber of the experimental group and the control group;
FIG. 9 illustrates the measurements of the change in the concentration of ranibizumab in plasma of the experimental group and the control group;
FIG. 10A illustrates the first day of culturing LB medium bacteria for the experimental group and the control group;
FIG. 10B illustrates the third day of culturing LB medium bacteria for the experimental group and the control group;
FIG. 11 illustrates the quantitative results of ELISA for inflammatory factors in the vitreous humor tissue;
FIG. 12A is a diagram illustrating the formation of a hyaluronic acid microgel using HA-NHS and HA-NH₂;
FIG. 12B is a diagram illustrating the formation of a hyaluronic acid microgel using HA-TA;
FIG. 12C is a diagram illustrating the formation of a hyaluronic acid microgel using HA-Mal and HA-SH;
FIG. 12D is a diagram illustrating the formation of a hyaluronic acid microgel using HA-VS and HA-SH;
FIG. 13 is a graph illustrating the measurements of the drug release behavior of a drug-loaded hyaluronic acid microgel;
FIG. 14 is a diagram illustrating the synthesis of a GPT copolymer;
FIG. 15A is a diagram illustrating the preparation of a GPT hydrogel;
FIG. 15B is a diagram illustrating the formation of a GPT hydrogel in which drug-loaded hyaluronic acid microgels are distributed;
FIG. 16 is the ¹H NMR spectrum and UV analysis results of the GPT copolymer;
FIG. 17 illustrates the change in the gelation time of the GPT hydrogel according to the change in the HRP concentration;
FIG. 18 is a graph measuring the mechanical strength of the GPT hydrogel over time;
FIG. 19 is an SEM image illustrating an internal structure of the GPT hydrogel;
FIG. 20 is a graph illustrating the measurements the drug release behavior of the GPT hydrogel carrying drug-loaded hyaluronic acid microgels;
FIG. 21 illustrates the measurements results of the change in the concentration of ranibizumab in vitreous humor of the experimental group and the control group;
FIG. 22 illustrates the measurements of the change in the concentration of ranibizumab in vitreous humor to retina of the experimental group and the control group;
FIG. 23 illustrates the measurements of the change in the concentration of ranibizumab in anterior chamber of the experimental group and the control group;
FIG. 24 illustrates the measurements of the change in the concentration of ranibizumab in plasma of the experimental group and the control group;
FIG. 25A illustrates the first day of culturing LB medium bacteria for the experimental group and the control group;
FIG. 25B illustrates the third day of culturing LB medium bacteria for the experimental group and the control group;
FIG. 26 illustrates the quantitative results of ELISA for inflammatory factors in the vitreous humor tissue;
FIG. 27A is a diagram illustrating the preparation of a GH hydrogel;
FIG. 27B is a diagram illustrating the formation of the GH hydrogel in which the drug-loaded hyaluronic acid microgels are distributed;
FIG. 28 is the ¹H NMR spectrum and UV analysis results of the GH copolymer;
FIG. 29 is a graph illustrating the measurements the drug release behavior of the GH hydrogel carrying the drug-loaded hyaluronic acid microgels;
FIG. 30A is a diagram of the preparation of a HA-VS/HA-SH hydrogel;
FIG. 30B is a diagram illustrating the formation of the HA-VS/HA-SH hydrogel in which the drug-loaded hyaluronic acid microgels are distributed;
FIG. 31 is the ¹H NMR spectrum and UV analysis results of the HA-VS/HA-SH copolymer; and
FIG. 32 is a graph illustrating the measurements the drug release behavior of the HA-VS/HA-SH hydrogel carrying the drug-loaded hyaluronic acid microgels.

### Best Mode

### Mode for Invention

The advantages and features of the present invention and methods of accomplishing the same may be understood more readily with reference to the following detailed description of examples and the accompanying drawings. These examples are provided so that this disclosure will be thorough and complete and will fully convey the concept of the invention to those skilled in the art, and the present invention will only be defined by the appended claims. Throughout the specification, the same reference numerals refer to the same components.

The present invention relates to a drug delivery system for injectable intraocular of a protein therapeutic agent or a drug for treating eye diseases, and may be implemented as a microgel and a hydrogel comprising the same. For example, a drug applicable to the present invention may be one or more selected from an injectable intraocular protein therapeutic agent, a chemical drug, a small molecule therapeutic, a gene, and a cell. Here, the protein therapeutic agent may consist of anti-vascular endothelial growth factors used for macular degeneration and diabetic retinopathy, etc., that is anti-VEGF (ranibizumab, aflibercept, bevacizumab, brolucizumab, etc.), rituximab or infliximab, etc., the chemical drug may consist of dexamethasone, triamcinolone, ganciclovir, methotrexate or vancomycin, etc., the small molecule therapeutic may consist of sugars, lipids, amino acids, fatty acids, phenolic compounds or alkaloids, etc., the gene may consist of siRNA(anti-VEGF), etc., and the cell may consist of retinal and intraocular cells such as RPE, photoreceptor, etc. and stem cell, etc. Hereinafter, examples using ranibizumab as a drug is described, but the present invention is not limited to such drug and may comprise any ophthalmic protein therapeutic agents, chemical drugs, small molecule therapeutics, genes and cells, etc.

The injectable intraocular microgel may be formed in a form in which a drug is loaded on a hyaluronic acid microgel generated by cross-linking a hyaluronic acid copolymer through a w/o emulsion method. Here, the hyaluronic acid copolymer may be at least one selected from the group consisting of hyaluronic acid vinylsulfone (HA-VS), hyaluronic acid-(N-hydroxy succinimide) (HA-NHS), hyaluronic acid-tyramine (HA-TA), and hyaluronic acid-maleimide (HA-Mal).

In addition, the injectable intraocular hydrogel of the present invention may consist of any hydrogel in which drug-loaded hyaluronic acid microgels are distributed. In the present invention, a GPT hydrogel, a GH hydrogel, and a HA-VA/HA-SH hydrogel are described as examples of the injectable intraocular hydrogels.

### Example 1. Synthesis of Hyaluronic Acid Microgel

### <1-1> Synthesis of Hyaluronic Acid Vinylsulfone (HA-VS) Copolymer

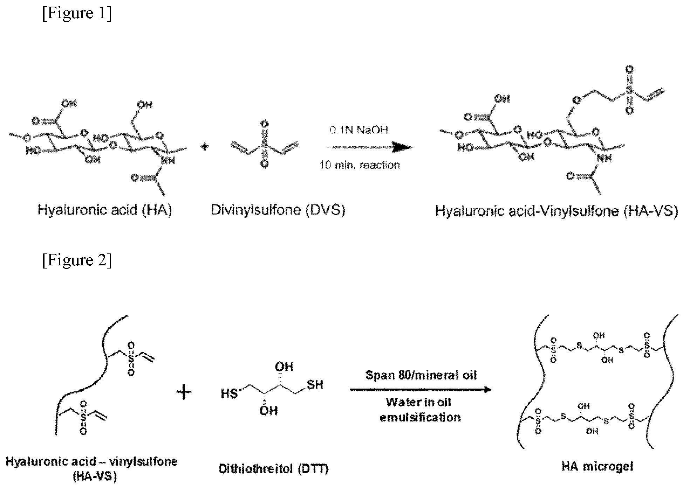

70 mmol of divinylsulfone (DVS) was added to a solution with 0.1 mmol/ml of -OH obtained by mixing 1 g of 4 kDa hyaluronic acid (HA) in 100 ml of 0.1 M sodium hydroxide solution, followed by reaction for 10 minutes. Using the bonding reaction of the vinyl group of divinylsulfone (DVS) and the hydroxy group of hyaluronic acid (HA), a hyaluronic acid-vinylsulfone (HA-VS) copolymer in which vinylsulfone (VS) is bonded to hyaluronic acid (HA) was obtained. The OH molar ratio of divinylsulfone:hyaluronic acid was 7:1. After synthesis, 5M HC1 was used for titration to pH 5 so that the reaction can end. After membrane dialysis (6000-8000 Da molecular weight blocking) was performed for 3 days, a hyaluronic acid vinylsulfone (HA-VA) copolymer was obtained through freeze drying. FIG. 1 is a diagram a process of reacting hyaluronic acid (HA) and divninylsulfone (DVS).

### <1-2> Preparation of Hyaluronic Acid Microgel (HA microgel)

8 wt% of the synthesized hyaluronic acid vinylsulfone (HA-VS) copolymer and dithiothreitol (DTT) were cross-linked through a thiol-ene reaction. The hyaluronic acid vinylsulfone (HA-VS) copolymer, the dithiothreitol (DTT), and a drug (10-fold concentrated ranibizumab) were added and mixed with 50 ml of mineral oil through a water in oil (w/o) emulsion method. The mixture was then dispersed in a hyaluronic acid microgel (HA microgel), using 2 ml of emulsifier (Span 80) and reacted 24 hours. After the reaction was complete, the resultant was put into 100 ml of isopropyl alcohol, and the mineral oil layer was removed. Next, the microgels were separated through centrifugation. After the separation, the microgels were redispersed in du-ionized water (DIW) to obtain drug-loaded hyaluronic acid microgels (HA microgels) through freeze-drying. FIG. 2 is a schematic diagram illustrating the formation of a hyaluronic acid microgel from HA-VS and DTT. The hyaluronic acid microgel shown in FIG. 2 has a structure in which a hyaluronic acid copolymer is cross-linked by a cross-linking functional group represented by Chemical Formula 1-1 below.

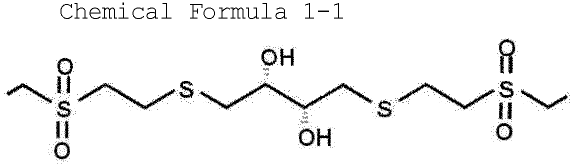

### Experimental Example 1. Evaluation of Physical Properties of Hyaluronic Acid Microgel

### <1-1> Analysis of Chemical Structure of HA-VS Copolymer and the Amount of Vinylsulfone (VS) Introduced through ¹H NMR

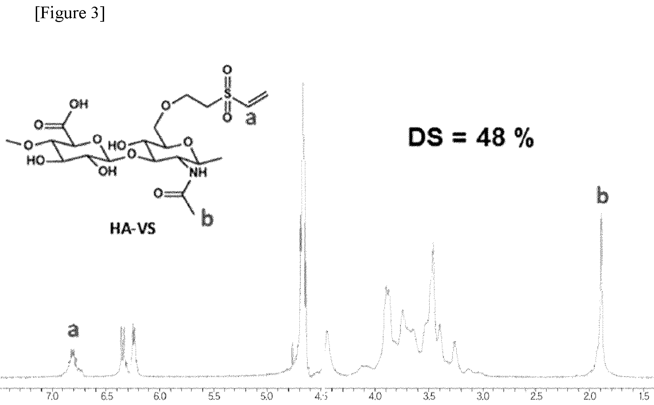

FIG. 3 is a ¹H NMR spectrum of a hyaluronic acid vinylsulfone (HA-VS) copolymer. It was confirmed that the synthesis was well performed by the peak points a and b of the vinylsulfone (VS) substituent. The degree of substitution (DS) of vinylsulfone (VS) was confirmed to be 48%.

### <1-2> Analysis of Size of Hyaluronic Acid Microgel

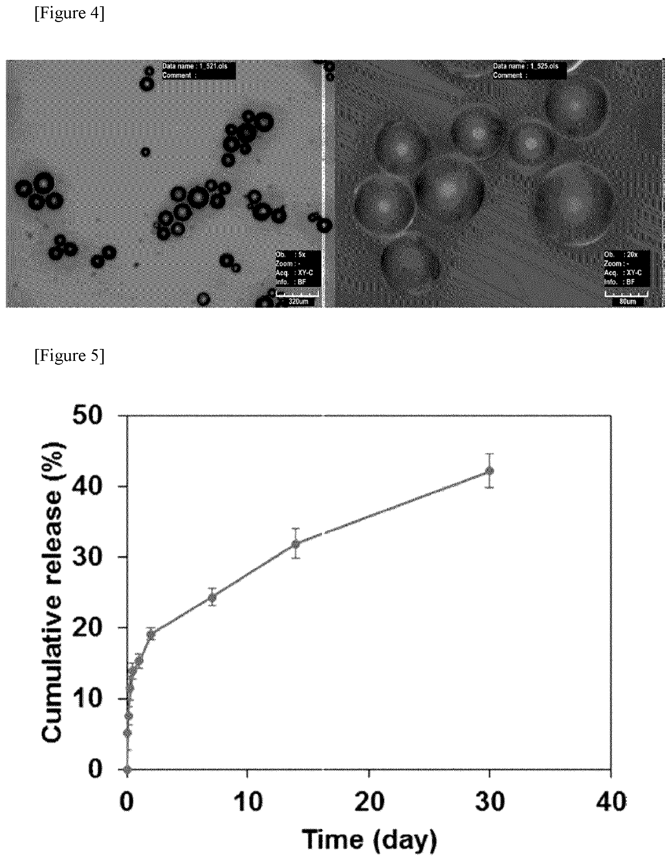

As a result of analyzing the hyaluronic acid microgel using a confocal laser scanning microscopy (CLSM), it was confirmed that the injectable intraocular microgels have an average size of 30 to 160 µm. FIG. 4 is a CLSM image of a hyaluronic acid microgel.

### <1-3> Evaluation of Drug Release Behavior of Hyaluronic Acid Microgel

Drug release of 8 mg of drug-loaded hyaluronic acid microgels was induced in DPBS. The release time interval was 1 hour, 3 hours, 6 hours, 12 hours, 1 day, 3 days, 7 days, 14 days, and 30 days, and the released drugs were quantified through enzyme immunoassay (ELISA). FIG. 5 is a graph illustrating the measurements of the drug release behavior of a drug-loaded hyaluronic acid microgel. It was confirmed that the drug release behavior of the hyaluronic acid microgels was released in a sustained release form for more than 30 days.

### <1-4> Evaluation of in vivo Pharmacokinetics through Animal Experiments

Pharmacokinetics was evaluated for the experimental group in which the hyaluronic acid microgel loaded with a drug (10-fold concentrated ranibizumab) was injected into a rabbit eye. In addition, the drug was injected into the rabbit eye without using a hyaluronic acid microgel carrier and evaluated as a control group. The control group consisted of a control group 1 injected with ranibizumab as a drug and control 2 injected with a 10-fold concentrated ranibizumab as a drug. The evaluation of in vivo pharmacokinetic was performed in the following manner.

After mixing zoletil (0.3 cc/kg) and rompun (0.25 cc/kg) as an anesthetic and anesthetizing the rabbit by intramuscular injection, the hyaluronic acid microgel and the control drug were injected into the rabbit eye in the range of 30-50 µ1. After anesthesia in the same manner for each planned period (1 hour, 1 day, 4 days, 8 days, 14 days, 30 days, 60 days, and 90 days), blood was collected, and after sacrificing the rabbit, the eyeballs were extracted and stored frozen. The collected blood was centrifuged to obtain a plasma sample and stored frozen.

The extracted eyeballs were separated into anterior chamber, vitreous humor, and retina tissue, and then lysate was prepared through lysis buffer treatment. For plasma and intraocular tissues, the dilution rate was set and the ranibizumab concentration in the tissue was measured through ranibizumab quantitative enzyme immunoassay (ELISA).

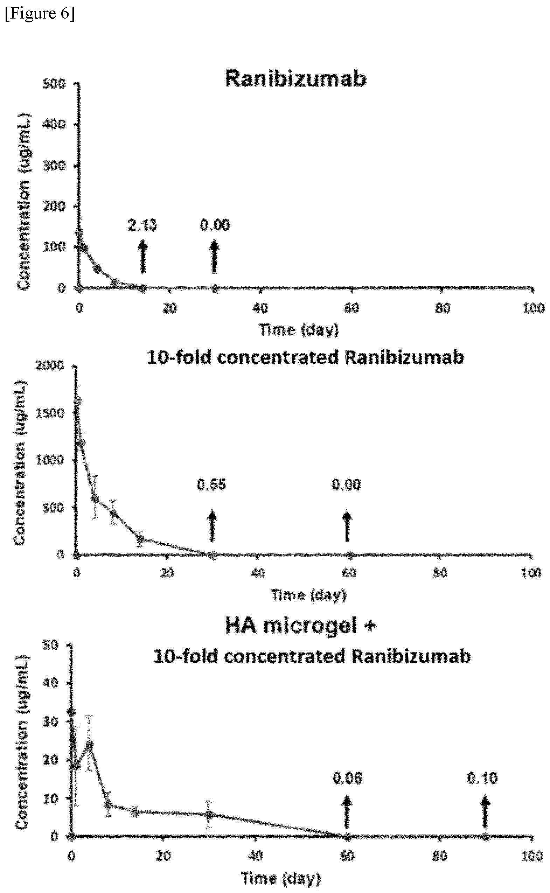

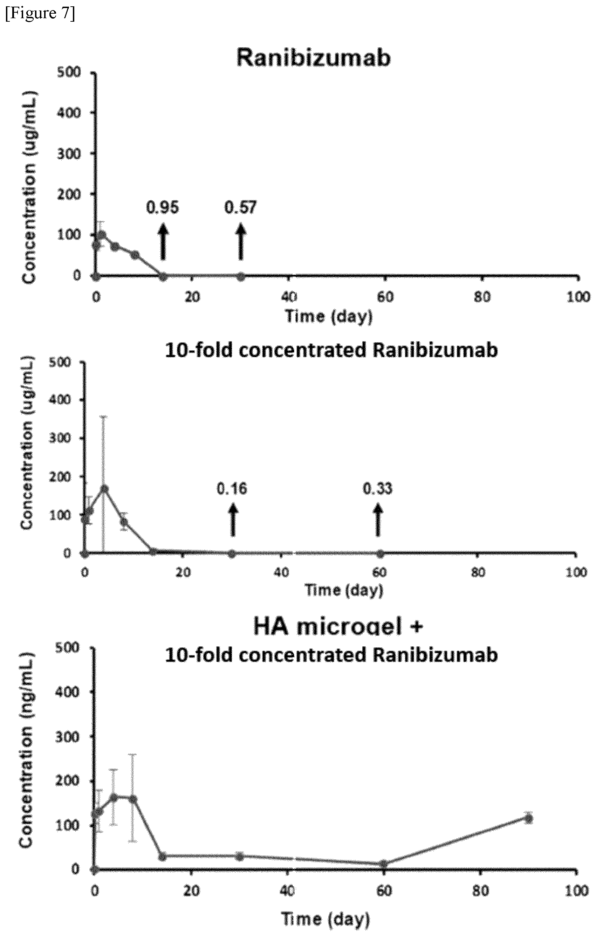

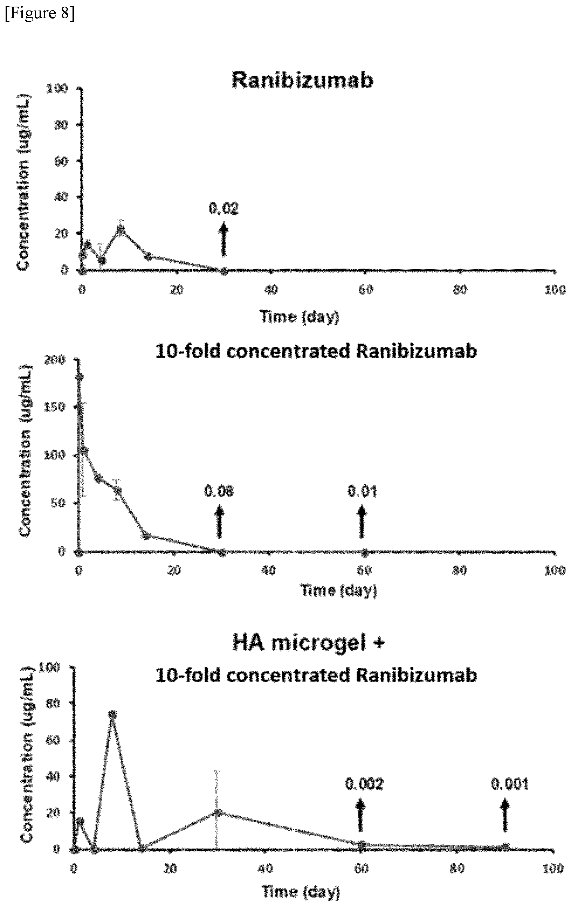

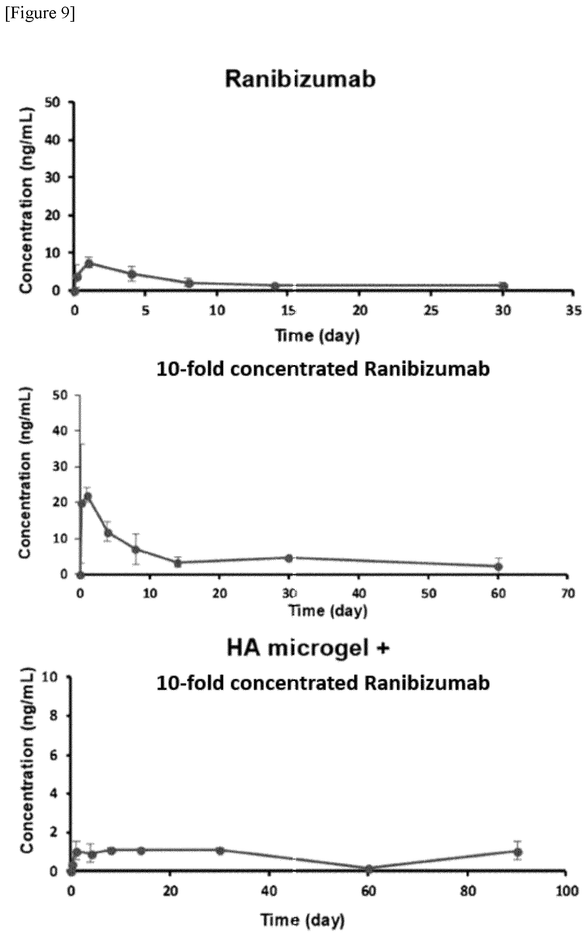

FIG. 6 illustrates measurement results of the change in the concentration of ranibizumab in vitreous humor of the experimental group and the control group. FIG. 7 illustrates the measurements of the change in the concentration of ranibizumab in vitreous humor to retina of the experimental group and the control group. FIG. 8 illustrates the measurements of the change in the concentration of ranibizumab in anterior chamber of the experimental group and the control group. FIG. 9 illustrates the measurements of the change in the concentration of ranibizumab in plasma of the experimental group and the control group. As shown in FIGs. 6 to 9, as a result of ELISA analysis, it was confirmed that the experimental group in which a hyaluronic acid microgel was injected in the vitreous humor, retina, anterior chamber, and plasma had a longer release behavior than the control group.

### <1-5> Evaluation of in vivo Safety of Drug and Formulation (Evaluation of Bacterial Infection and Quantitative Evaluation of Infection Factors)

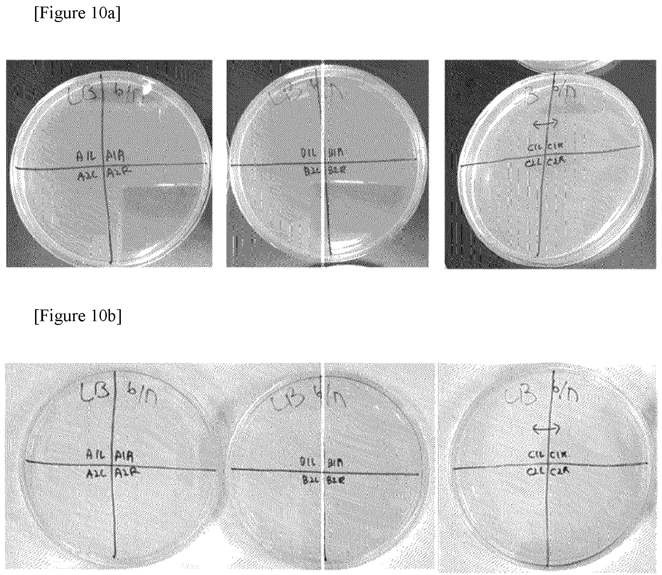

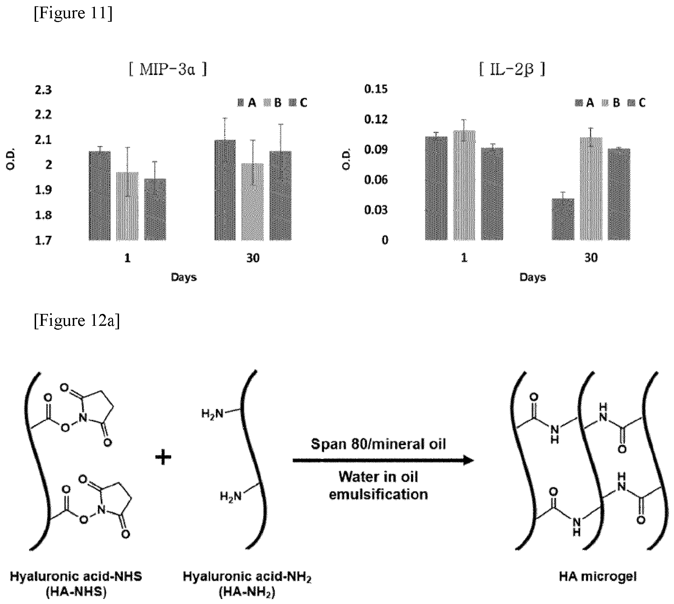

In order to check for infection and inflammation of the hyaluronic acid microgel formulations injected into the eye, bacterium were cultured in LB medium for control group 1 (ranibizumab injected group), control group 2 (10-fold concentrated ranibizumab injected group), and the experimental group (hyaluronic acid microgel injected group) and inflammatory factor (MIP-3α and IL-2β) quantitative ELISA was performed on the 1^{st} and 3^{rd} days for the vitreous humor tissue. FIG. 10A illustrates the first day of culturing LB medium bacteria for the experimental group and the control group, and FIG. 10B illustrates the third day of culturing LB medium bacteria for the experimental group and the control group (From the left, control group 1, control group 2, and experimental group are shown). FIG. 11 illustrates the quantitative results of ELISA for inflammatory factors in the vitreous humor tissue (left = MIP-3α, right = IL-2β, A = control group 1, B = control group 2, and C = experimental group).

As shown in FIGs. 10A to 11, infection and inflammation were not observed in the two experiments.

### Example 2. Preparation and Evaluation of Hyaluronic Acid Microgel Using Different Cross-linking Methods

Besides the method explained in Example 1, a hyaluronic acid microgel which was cross-linked by the NHS-NH₂ cross-linking reaction of 8 wt% of hyaluronic acid-(N-hydroxysuccinimide) (HA-NHS)/hyaluronic acid-amine (NHS-NH₂), enzymatic cross-linking reaction (HRP/H₂O₂) of hyaluronic acid-tyramine (HA-TA), thiol-maleimide cross-linking reaction of hyaluronic acid-maleimide (HA-Mal)/hyaluronic acid-thiol (HA-SH), and thiol-ene reaction of hyaluronic acid vinylsulfone (HA-VS)/hyaluronic acid thiol (HA-SH), and on which a drug is carried through a water in oil (w/o) emulsion method, was prepared.

FIG. 12A is a diagram illustrating the formation of a hyaluronic acid microgel using HA-NHS and HA-NH₂. The hyaluronic acid microgel shown in FIG. 12A has a structure in which the hyaluronic acid copolymer is cross-linked by a cross-linking functional group represented by Chemical Formula 1-2 below.

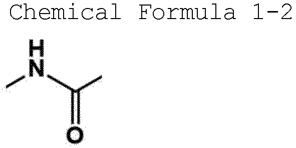

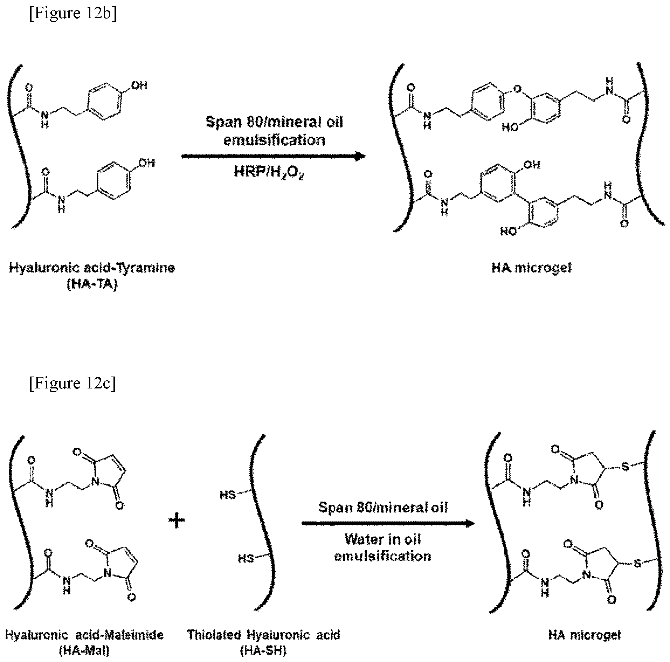

FIG. 12B is a diagram illustrating the formation of a hyaluronic acid microgel using HA-TA. The hyaluronic acid microgel shown in FIG. 12B has a structure in which the hyaluronic acid copolymer is cross-linked by a cross-linking functional group represented by Chemical Formula 1-3 and/or Chemical Formula 1-4 below.

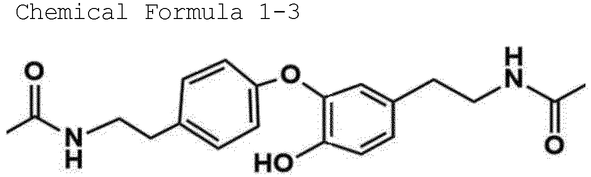

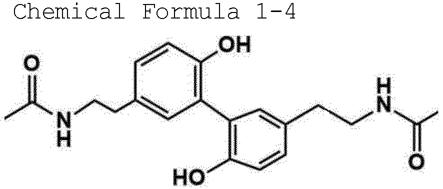

FIG. 12C is a diagram illustrating the formation of a hyaluronic acid microgel using HA-Mal and HA-SH. The hyaluronic acid microgel shown in FIG. 12C has a structure in which the hyaluronic acid copolymer is cross-linked by a cross-linking functional group represented by Chemical Formula 1-5 below.

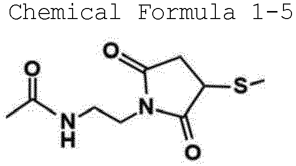

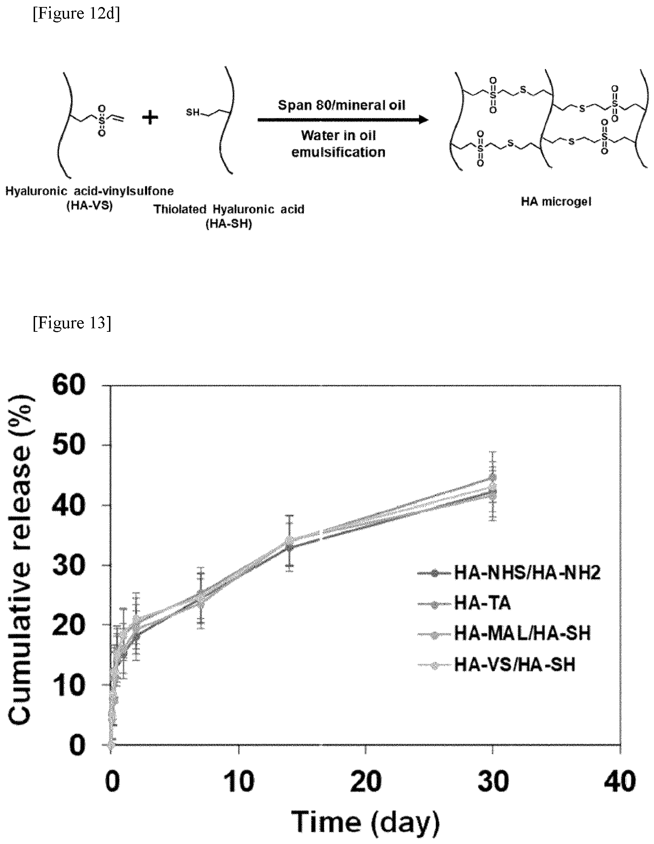

FIG. 12D is a diagram illustrating the formation of a hyaluronic acid microgel using HA-VS and HA-SH. The hyaluronic acid microgel shown in FIG. 12D has a structure in which the hyaluronic acid copolymer is cross-linked by a cross-linking functional group represented by Chemical Formula 1-6 below.

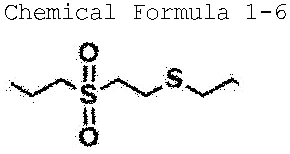

The drug release behavior of hyaluronic acid microgels prepared through these different cross-linking methods was reviewed. Drug release of 8 mg of drug-loaded hyaluronic acid microgels was induced in DPBS. The release time interval was 1 hour, 3 hours, 6 hours, 12 hours, 1 day, 3 days, 7 days, 14 days, and 30 days, and the released drugs were quantified through enzyme immunoassay (ELISA). FIG. 13 is a graph illustrating the measurements of the drug release behavior of a drug-loaded hyaluronic acid microgel. It was confirmed that the drug release behavior of the hyaluronic acid microgels prepared according to each cross-linking method was released in a sustained release form for more than 30 days.

As reviewed above, various cross-linking methods may be applied to prepare the hyaluronic acid microgel of the present invention. However, the present invention is not limited thereto, and hyaluronic acid microgels can be formed through other cross-linking reactions.

### Example 3. Synthesis of GPT Hydrogel

### <3-1> Synthesis of GPT Copolymer

### (a) Synthesis of Polyethylene Glycol-4-nitrophenylchloroformate (PEG-PNC)

After dissolving 10 g (2.9 mmol) of polyethylene glycol (PEG) in 100 ml of methylene chloride (MC), in this solution, a solution of 0.779 g (6.38 mmol) of 4-dimethylaminopyridine (DMAP) and 0.645 g (6.38 mmol) of triethylamine (TEA) dissolved in 10 ml of methylene chloride (MC) and a solution of 1.286 g (6.38 mmol) of 4-nitrophenylchloroformate (PNC) dissolved in 50 mL of methylene chloride (MC) were sequentially mixed. At this time, the molar ratio of PEG:DMAP:TEA:PNC was 1:2.2:2.2:2.2, the reaction temperature was 30°C, and the reaction was carried out in a nitrogen atmosphere for 24 hours.

After completion of the reaction, the reagents remaining in the solution were removed using a filter, and then the reaction solution was concentrated using a rotary evaporation concentrator. The concentrated solution was added dropwise to 1800 ml of cold ether to generate a precipitate, and the precipitate was filtered using a filter to obtain a product. The obtained product was left in a vacuum oven for 24 hours to remove the residual organic solvent, and as a result, polyethylene glycol-4-nitrophenylchloroformate (PEG-PNC), which is a white powdery product, was obtained.

### (b) Synthesis of GPT Copolymer

The reaction was carried out by adding a solution obtained by dissolving 0.202 g (1.471 mmol) of tyramine (TA) in 50 ml of DMSO to a solution obtained by dissolving 5 g (1.471 mmol) of PEG-PNC in 100 ml of dimethylsulfoxide (DMSO). The molar ratio of PEG-PNC:TA was 1:1. The reaction temperature was 30°C, and the reaction was carried out in a nitrogen atmosphere for 6 hours. After the reaction, a gelatin solution (1 g/200 ml in DMSO) was mixed and the reaction was performed at 40°C in a nitrogen atmosphere for 24 hours.

After completion of the reaction, unreacted PEG-TA was removed from the reaction solution through membrane dialysis (6000-8000 Da molecular weight blocking) using distilled water. After the dialysis was completed, the solution was freeze-dried to obtain a gelatin-PEG-TA (GPT) copolymer.

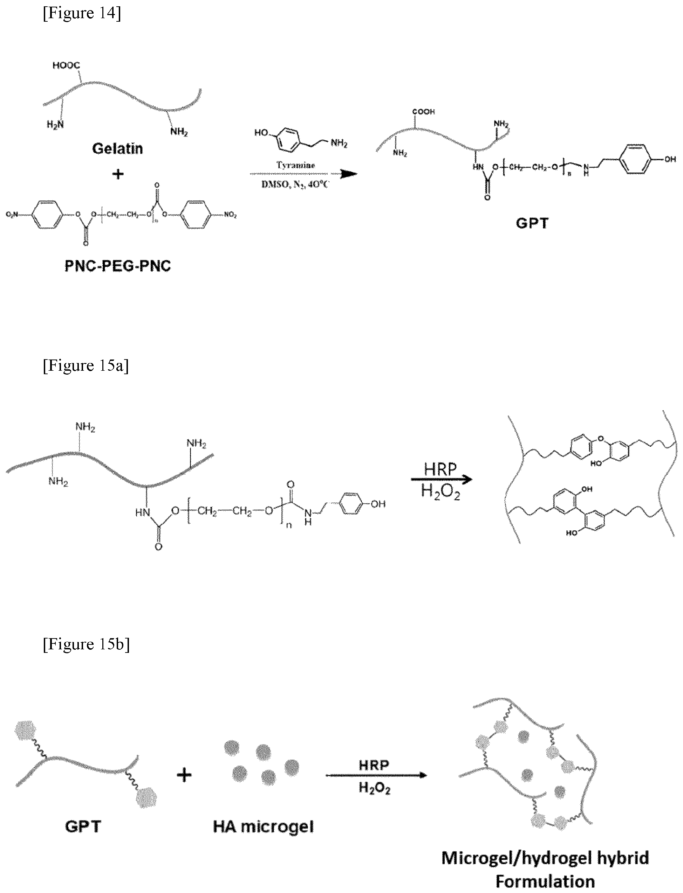

FIG. 14 is a diagram illustrating the synthesis of a GPT copolymer.

### <3-2> Preparation of GPT Hydrogel

A GPT hydrogel was formed by adding horseradish peroxidase (HRP) and hydrogen peroxide (H₂O₂) to the synthesized gelatin derivative, i.e., the GPT copolymer, and cross-linking the phenol derivatives in the GPT copolymer with each other. Here, the GPT hydrogel can control the gelation time or mechanical strength by controlling the concentration of horseradish peroxidase (HRP) or hydrogen peroxide. FIG. 15A is a diagram illustrating the preparation of a GPT hydrogel. The hydrogel shown in FIG. 15A has a structure that is cross-linked by a cross-linking functional group represented by Chemical Formula 2-1 and/or Chemical Formula 2-2 below.

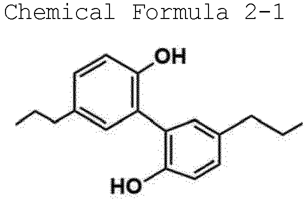

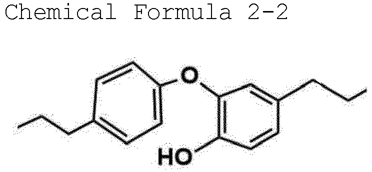

A composite formation, i.e., an injectable intraocular hydrogel, in which drug-loaded hyaluronic acid microgels are distributed in the GPT hydrogel, is prepared by mixing hyaluronic acid microgel loaded with a drug (10-fold concentrated ranibizumab) with 150 µl of a GPT copolymer dissolved in a horseradish peroxidase (HRP) solution and 150 µl of a GPT copolymer dissolved in a hydrogen peroxide solution. FIG. 15B is a diagram illustrating the formation of a GPT hydrogel in which drug-loaded hyaluronic acid microgels are distributed.

### Experimental Example 3. Evaluation of Physical Properties of GPT Hydrogel

### <3-1> Analysis of Chemical Structure of GPT Copolymers through ¹H NMR

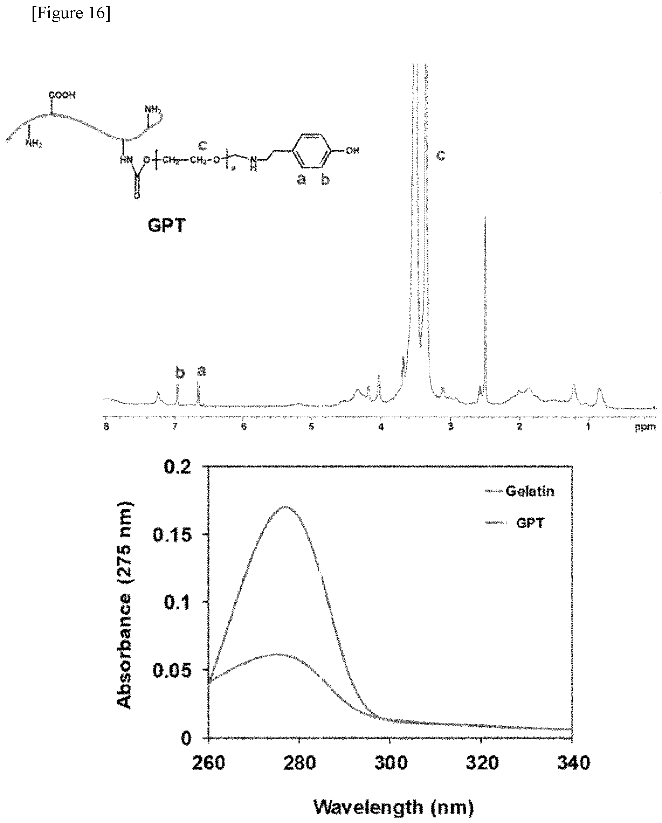

FIG. 16 is the ¹H NMR spectrum and UV analysis results of the GPT copolymer. It was confirmed that the synthesis was well performed by the peak points a, b, and c of the GPT copolymer and it was confirmed that 280.17 µmol per g of phenol gelatin derivative, which is a cross-linking molecule, was introduced.

### <3-2> Control of Gelation Time and Mechanical Strength of GPT Hydrogel

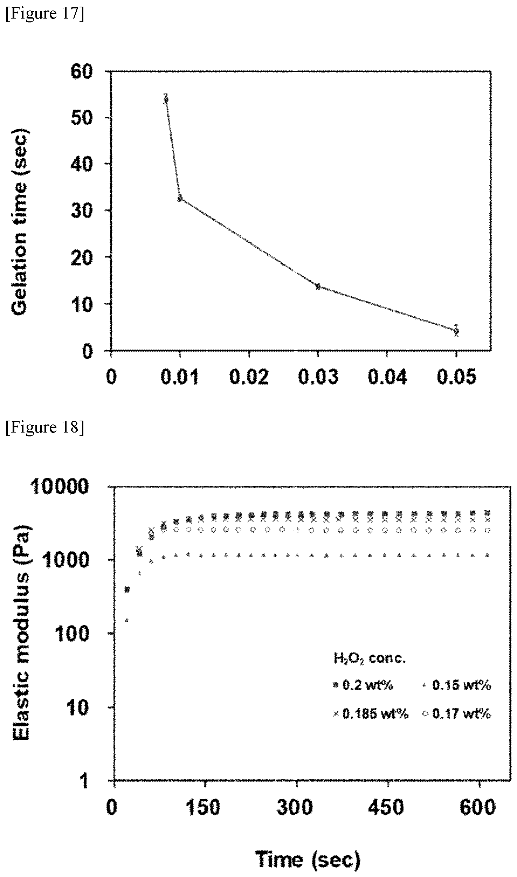

GPT hydrogel can control the gelation time according to the concentration of horseradish peroxidase (HRP) and hydrogen peroxide. In addition, the gelation time can be adjusted from a minimum of 2 seconds to a maximum of 2 minutes according to each element. That is, as the concentration of HRP increases, the gelation time becomes shorter, and as the concentration of HRP increases, the decomposition of hydrogen peroxide is accelerated, and eventually the generation rate of radical becomes faster. Therefore, the gelation time is accelerated because the gel is formed by the generated radical. FIG. 17 illustrates the change in the gelation time of the GPT hydrogel according to the change in the HRP concentration.

The mechanical strength can be adjusted according to the concentration of hydrogen peroxide. If the concentration of hydrogen peroxide is adjusted from 0.15 wt% to 0.2 wt%, the mechanical strength is adjusted from 800 Pa to 7000 Pa. The adjustment of mechanical strength is a phenomenon that occurs when the cross-linking density of hydrogel is adjusted, and the drug inside hydrogel is slowly released as the cross-linking density increases, which is confirmed using a rheometer. FIG. 18 is a graph measuring the mechanical strength of the GPT hydrogel over time.

### <3-3> Analysis of the Internal Structure of GPT Hydrogel through SEM Analysis

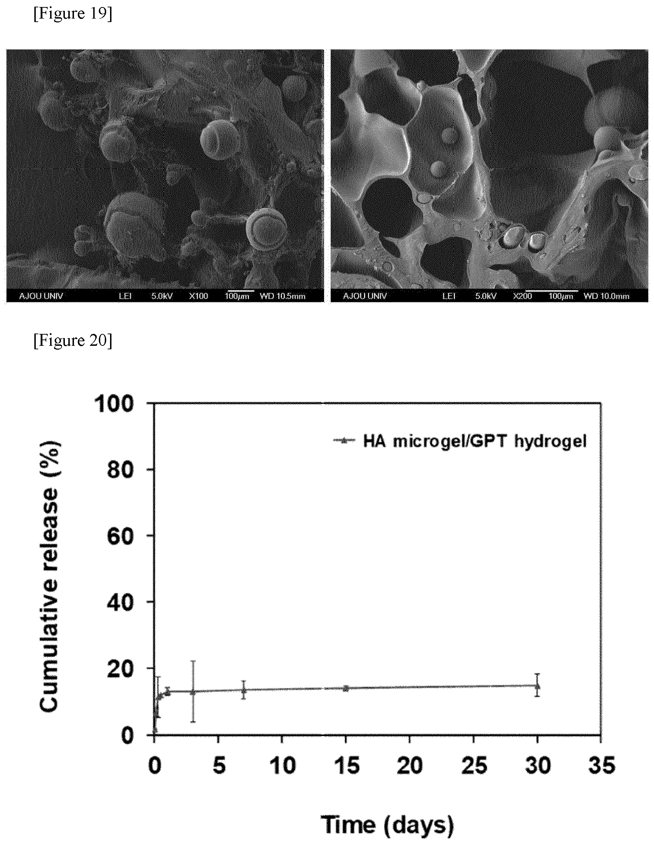

FIG. 19 is an SEM image illustrating an internal structure of the GPT hydrogel. It was confirmed that the hyaluronic acid microgels were well distributed in a GPT hydrogel.

### <3-4> Evaluation of Drug Release Behavior of GPT Hydrogel

After preparing a composite formulation by carrying drug-loaded hyaluronic acid microgels in a GPT hydrogel, drug release was induced in DPBS. The release time interval was 1 hour, 3 hours, 6 hours, 12 hours, 1 day, 3 days, 7 days, 14 days, and 30 days, and the released drugs were quantified through ELISA. FIG. 20 is a graph illustrating the measurements the drug release behavior of the GPT hydrogel carrying drug-loaded hyaluronic acid microgels. It was confirmed that the drug release behavior of the GPT hydrogel was slowly released for more than 30 days.

### <3-5> Evaluation of in vivo Pharmacokinetics through Animal Experiments

Pharmacokinetics was evaluated for the experimental group in which a composite formulation consisting of the hyaluronic acid microgel loaded with a drug (10-fold concentrated ranibizumab) and a GPT hydrogel was injected into a rabbit eye. In addition, the drug was injected into the rabbit eye without using the composite formulation of the hyaluronic acid microgel/GPT hydrogel and evaluated as a control group. The control group consisted of a control group 1 injected with ranibizumab as a drug and control 2 injected with a 10-fold concentrated ranibizumab as a drug. The evaluation of in vivo pharmacokinetic was performed in the following manner.

After mixing zoletil (0.3 cc/kg) and rompun (0.25 cc/kg) as an anesthetic and anesthetizing the rabbit by intramuscular injection, the composite formulation and the control drug were injected into the rabbit eye in the range of 30-50 µl. After anesthesia in the same manner for each planned period (1 hour, 1 day, 4 days, 8 days, 14 days, 30 days, 60 days, and 90 days), blood was collected, and after sacrificing the rabbit, the eyeballs were extracted and stored frozen. The collected blood was centrifuged to obtain a plasma sample and stored frozen.

The extracted eyeballs were separated into anterior chamber, vitreous humor, and retina tissue, and then lysate was prepared through lysis buffer treatment. For plasma and intraocular tissues, the dilution rate was set and the ranibizumab concentration in the tissue was measured through ranibizumab quantitative enzyme immunoassay (ELISA).

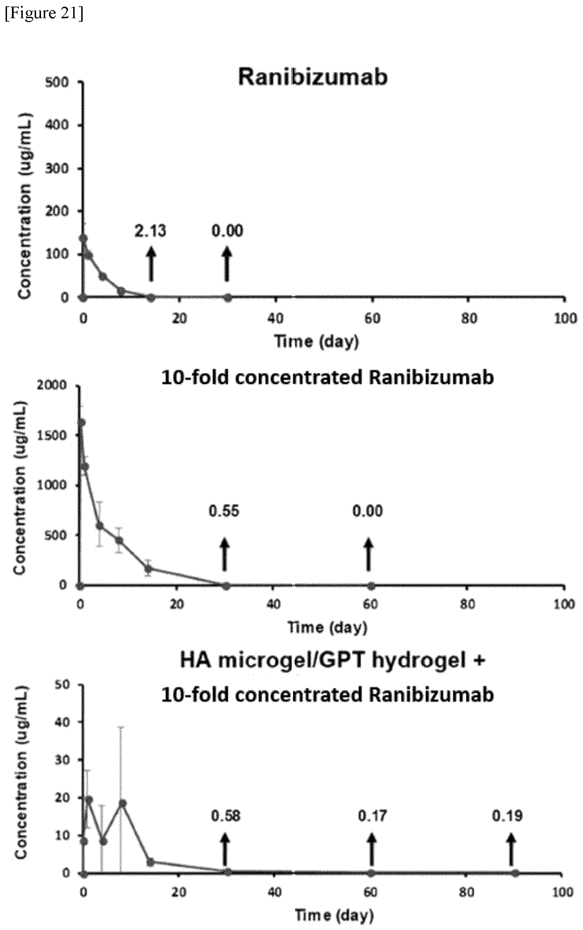

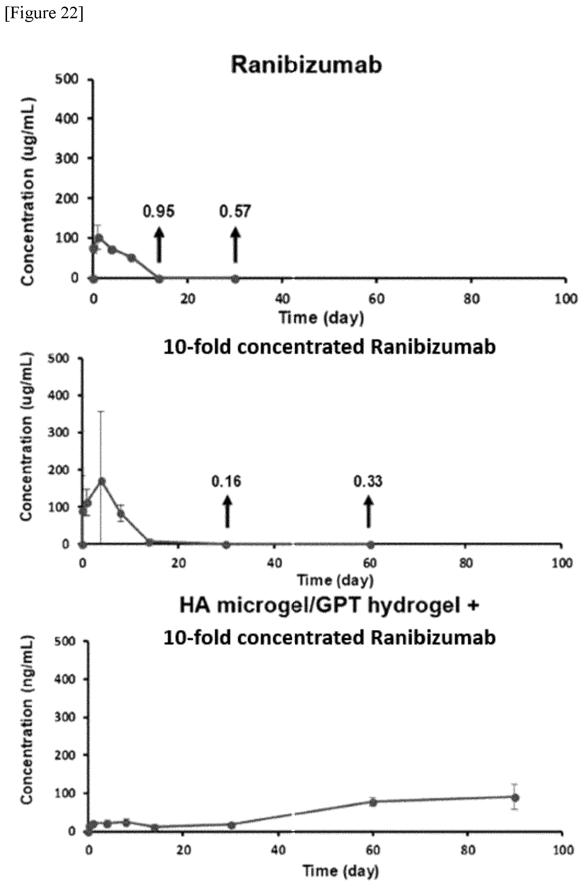

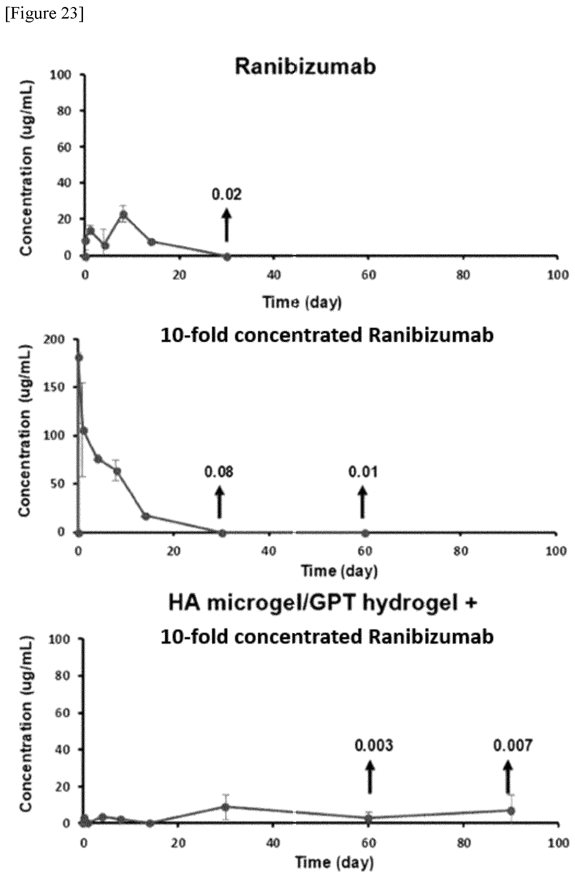

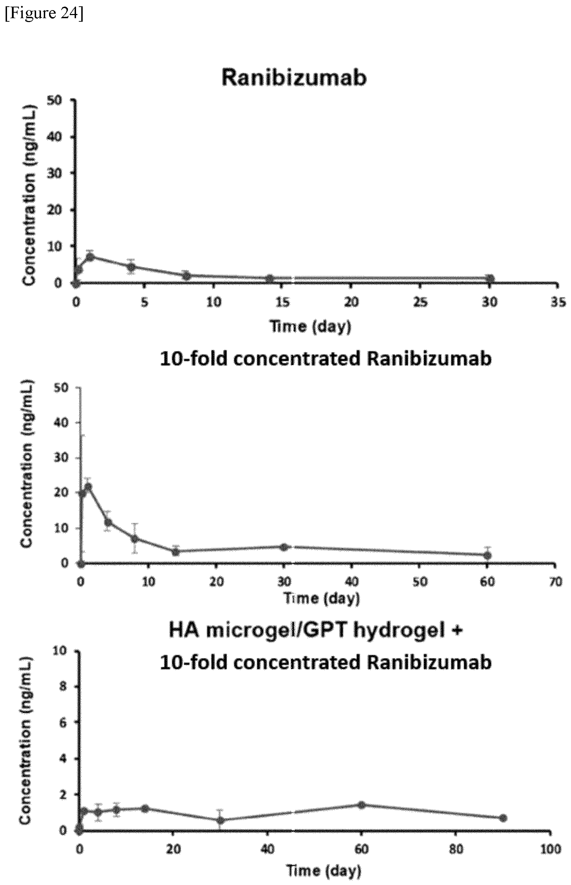

FIG. 21 illustrates the measurement results of the change in the concentration of ranibizumab in vitreous humor of the experimental group and the control group. FIG. 22 illustrates the measurements of the change in the concentration of ranibizumab in vitreous humor to retina of the experimental group and the control group. FIG. 23 illustrates the measurements of the change in the concentration of ranibizumab in anterior chamber of the experimental group and the control group. FIG. 24 illustrates the measurements of the change in the concentration of ranibizumab in plasma of the experimental group and the control group. As shown in FIGs. 21 to 24, as a result of ELISA analysis, it was confirmed that the experimental group in which the composite formulation of hyaluronic acid microgel/GPT hydrogel was injected in the vitreous humor, retina, anterior chamber, and plasma had a longer release behavior than the control group.

### <3-6> Evaluation of in vivo Safety of Drug and Formulation (Evaluation of Bacterial Infection and Quantitative Evaluation of Infection Factors)

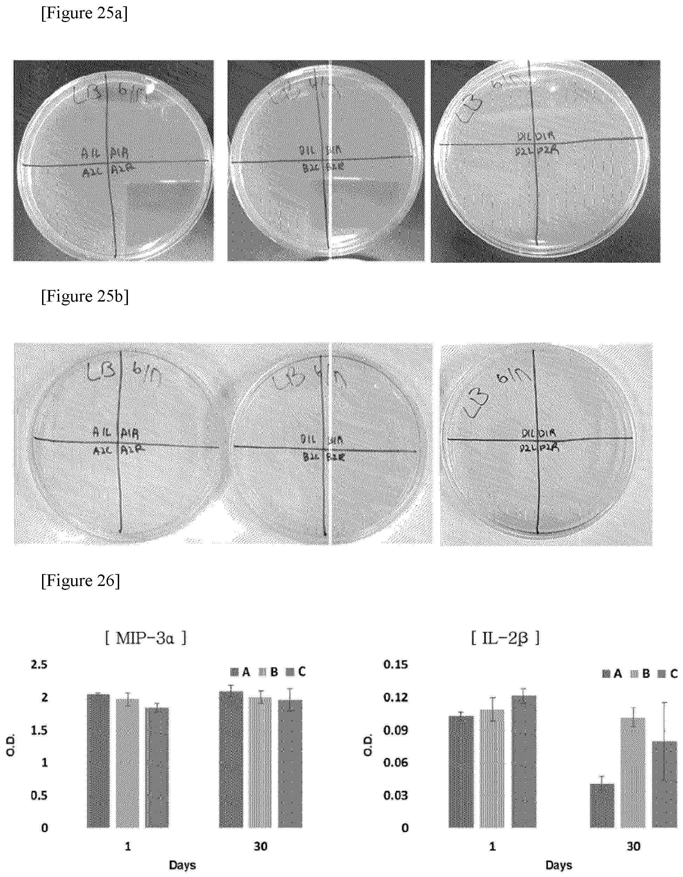

In order to check for infection and inflammation of the composite formulation of the hyaluronic acid microgel/GPT hydrogel injected into the eye, bacteria were cultured in LB medium for control group 1 (ranibizumab injected group), control group 2 (10-fold concentrated ranibizumab injected group), and the experimental group (composite formulation injected group) and inflammatory factor (MIP-3α and IL-2β) quantitative ELISA was performed on the 1^{st} and 3^{rd} days for the vitreous tissue. FIG. 25A shows the first day of culturing LB medium bacteria for the experimental group and the control group. FIG. 25b shows the third day of culturing LB medium bacteria for the experimental group and the control group (From the left, control group 1, control group 2, and experimental group are shown). FIG. 26 shows the quantitative results of ELISA for inflammatory factors in the vitreous humor tissue (left = MIP-3α, right = IL-2β, A = control group 1, B = control group 2, and C = experimental group).

As shown in FIGs. 25A to 26, infection and inflammation were not observed in the two experiments.

### Example 4. Synthesis of GH Hydrogel

### <4-1> Synthesis of GH Copolymer

10 g of gelatin was dissolved in 200 ml of 0.1M 2-(N-morpholino) ethanesulfonic acid (MES) (solution A). 0.609 g (4 mmol) of 4-hydroxyphenylacetic acid (HPA) was dissolved in 50 ml of 0.1M MES (solution B). 0.92 g (4.8 mmol) of 1-ethyl-3-(3-dimethylaminopropyl)-carbodiimide (EDC) and 0.276 g (2.4 mmol) of N-hydroxysuccinimide (NHS) were dissolved in 5 ml of 0.1M MES, respectively. Then, the EDC solution and NHS solution were sequentially added to the solution B at 15-minute intervals. Solution B activated by EDC/NHS was mixed with solution A to start the reaction. At this time, the reaction temperature was 40°C, and the reaction time was 24 hours.

After completion of the reaction, the reaction solution was filtered using a syringe filter (450 nm). Then, membrane dialysis (3500 da molecular weight blocking) was performed in distilled water. The dialysis-completed solution was freeze-dried to obtain a GH (gelatin-hydroxyphenylacetic acid) copolymer.

### <4-2> Preparation of GH Hydrogel

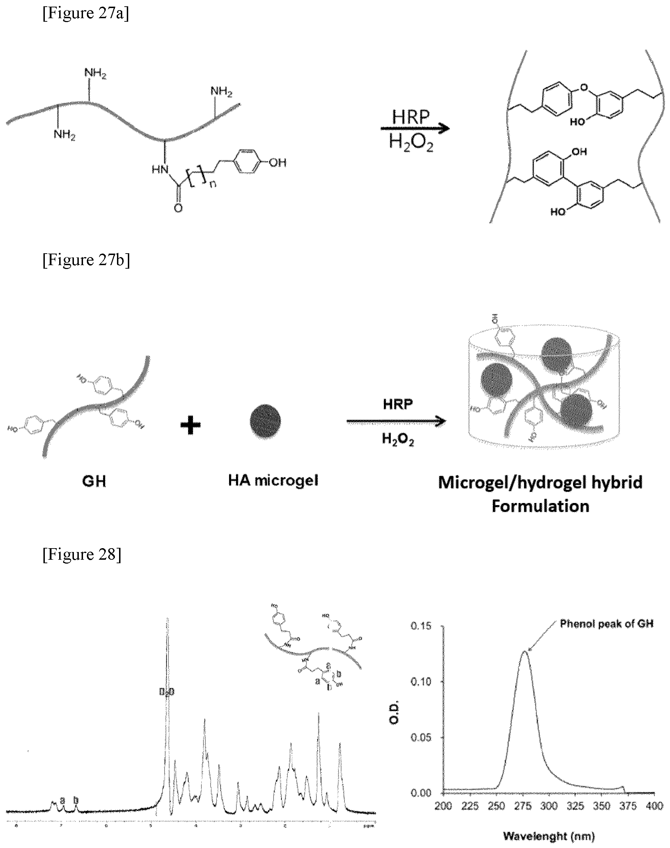

A GH hydrogel was formed by adding HRP and H₂O₂ to the synthesized GH copolymer and cross-linking the phenol derivatives in the GH copolymer with each other. FIG. 27A is a diagram illustrating the preparation of a GH hydrogel. The hydrogel shown in FIG. 27A has a structure cross-linked by a cross-linking functional group represented by Chemical Formula 2-1 and/or Chemical Formula (2-2) below.

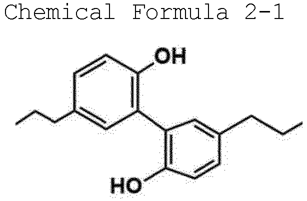

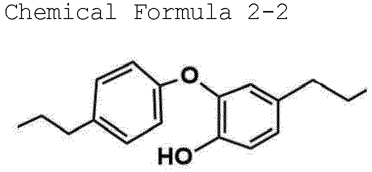

A composite formation, i.e., an injectable intraocular hydrogel, in which drug-loaded hyaluronic acid microgels are distributed in the GH hydrogel, is prepared by mixing hyaluronic acid microgel loaded with a drug (10-fold concentrated ranibizumab) with 150 µl of a GH copolymer dissolved in HRP solution and 150 µl of a GH copolymer dissolved in a hydrogen peroxide solution. FIG. 27B is a diagram illustrating the formation of the GH hydrogel in which the drug-loaded hyaluronic acid microgels are distributed.

### Experimental Example 4. Evaluation of Physical Properties of GH Hydrogel

### <4-1> Analysis of Chemical Structure of GH Copolymers through ¹H NMR

FIG. 28 is the ¹H NMR spectrum and UV analysis results of the GH copolymer. It was confirmed that the synthesis was well performed by the peak points a and b of the GH copolymer and it was confirmed that 153.8 µmol per GH g of phenol, which is a cross-linking molecule, was introduced.

### <4-2> Evaluation of Drug Release Behavior of GH Hydrogel

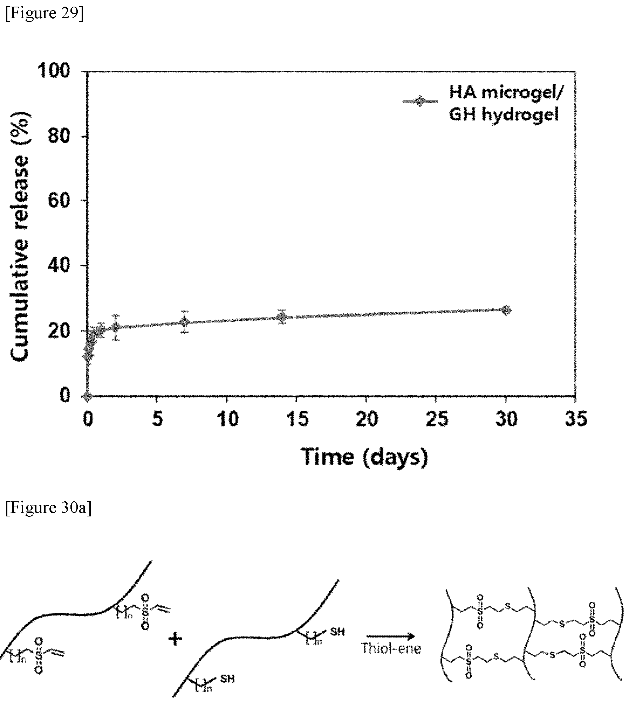

After preparing a composite formulation by carrying drug-loaded hyaluronic acid microgels in a GH hydrogel, drug release was induced in DPBS. The release time interval was 1 hour, 3 hours, 6 hours, 12 hours, 1 day, 3 days, 7 days, 14 days, and 30 days, and the released drugs were quantified through ELISA. FIG. 29 is a graph illustrating the measurements the drug release behavior of the GH hydrogel carrying the drug-loaded hyaluronic acid microgels. It was confirmed that the drug release behavior of the GH hydrogel was slowly released for more than 30 days.

### Example 5. Synthesis of HA-VS/HA-SH Hydrogel

### <5-1> Synthesis of HA-SH Copolymer

1 g of hyaluronic acid (HA) was dissolved in 80 ml of MES buffer (pH 5). 1.63 g (10.5 mmol) of EDC and 1.812 g (15.75 mmol) of NHS were dissolved in 20 ml of MES buffer, respectively. EDC solution and NHS solution were sequentially added to the hyaluronic acid solution at 15-minute intervals. 1.193 g (10.5 mmol) of Cysteamine HCL was added to the hyaluronic acid solution activated by EDC/NHS to start the reaction. At this time, the reaction temperature was a room temperature and the reaction time was 24 hours.

After completion of the reaction, membrane dialysis (6000-8000 Da molecular weight blocking) of the reaction solution was performed. The dialysate-completed solution was freeze-dried to obtain a thiolated hyaluronic acid (HA-SH) derivative.

### <5-2> Preparation of HA-VS/HA-SH Hydrogel

A HA-VS/HA-SH hydrogel was formed by mixing the synthesized HA-VS and HA-SH and cross-linking a vinyl group of HA-VS and a thiol group of HA-SH through thiol-ene reaction with each other. FIG. 30A is a diagram of the preparation of a HA-VS/HA-SH hydrogel. The hydrogel shown in FIG. 30A has a cross-linked structure by a cross-linking functional group represented by Chemical Formula 2-3 below.

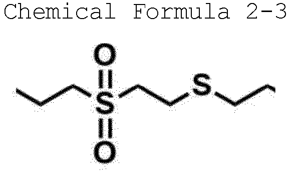

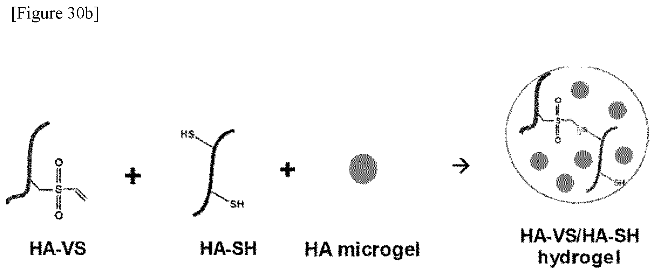

A composite formation, i.e., an injectable intraocular hydrogel, in which drug-loaded hyaluronic acid microgels are distributed in the HA-VS/HA-SH hydrogel, was prepared by mixing hyaluronic acid microgel loaded with a drug (10-fold concentrated ranibizumab) with 150 µl of a HA-SH solution and 150 µl of HA-VS solution. FIG. 30B is a diagram illustrating the formation of the HA-VS/HA-SH hydrogel in which the drug-loaded hyaluronic acid microgels are distributed.

### Experimental Example 5. Evaluation of Physical Properties of HA-VS/HA-SH Hydrogel

### <5-1> Analysis of Chemical Structure of HA-SH Copolymers through ¹H NMR

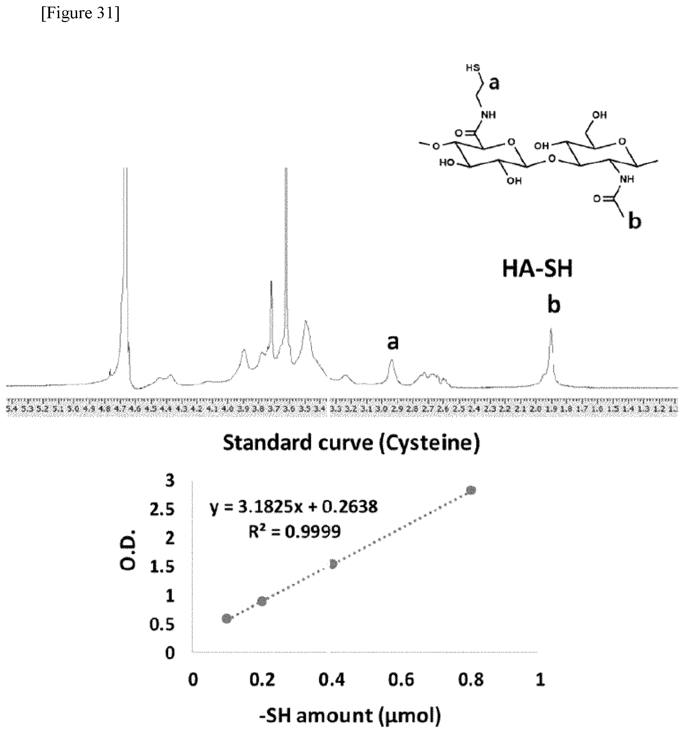

FIG. 31 is the ¹H NMR spectrum and UV analysis results of the HA-VS/HA-SH copolymer. It was confirmed that the synthesis was well performed by the peak points a and b of the HA-SH copolymer and it was confirmed that 230.5 µmol per HA-SH g of thiol, which is a cross-linking molecule, was introduced.

### <5-2> Evaluation of Drug Release Behavior of HA-VS/HA-SH Hydrogel

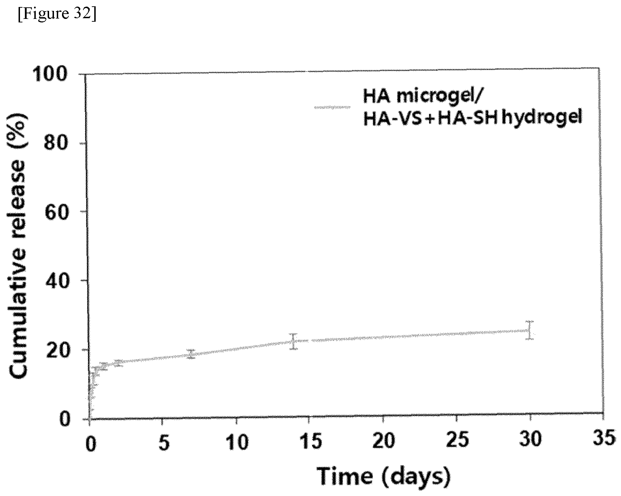

After preparing a composite formulation by carrying drug-loaded hyaluronic acid microgels in a HA-VS/HA-SH hydrogel, drug release was induced in DPBS. The release time interval was 1 hour, 3 hours, 6 hours, 12 hours, 1 day, 3 days, 7 days, 14 days, and 30 days, and the released drugs were quantified through ELISA. FIG. 32 is a graph illustrating the measurements the drug release behavior of the HA-VS/HA-SH hydrogel carrying the drug-loaded hyaluronic acid microgels. It was confirmed that the drug release behavior of the HA-VS/HA-SH hydrogel was slowly released for more than 30 days.

## Claims

1. A method of preparing an injectable intraocular hydrogel, the method comprising:
generating hyaluronic acid microgels by causing a hyaluronic acid copolymer to undergo a cross-linking reaction of a w/o emulsion method;
loading a drug on the hyaluronic acid microgels; and
distributing the hyaluronic acid microgels in the hydrogel.

2. The method of claim 1, wherein the generating of the hyaluronic acid microgels comprises:
synthesizing a hyaluronic acid vinylsulfone (HA-VS) copolymer by causing divinylsulfone (DVS) and hyaluronic acid (HA) to undergo a bonding reaction; and
causing the synthesized hyaluronic acid vinylsulfone (HA-VS) copolymer and dithiothreitol (DTT) to undergo a thiol-ene cross-linking reaction.

3. The method of claim 1, wherein the generating of the hyaluronic acid microgels is performed by NHS-NH2 cross-linking of hyaluronic acid-(N-hydroxy succinimide) (HA-NHS) and hyaluronic acid-amine (HA-NH₂).

4. The method of claim 1, wherein the generating of the hyaluronic acid microgels comprises:
adding horseradish peroxidase (HRP) and hydrogen peroxide (H₂O₂) to hyaluronic acid-tyramine (HA-TA); and
causing the resulting mixture to undergo an enzymatic cross-linking.

5. The method of claim 1, wherein the generating of the hyaluronic acid microgels is performed by thiol-ene cross-linking of hyaluronic acid vinylsulfone (HA-VS) and hyaluronic acid thiol (HA-SH).

6. The method of claim 1, comprising:
preparing a GPT hydrogel by adding horseradish peroxidase (HRP) and hydrogen peroxide to a gelatin-polyethylene glycol-tyramine (GPT) copolymer so that phenol derivatives in the GPT copolymer cross-link with each other; and
mixing the injectable intraocular microgel in the GPT hydrogel.

7. The method of claim 1, comprising:
preparing a GH hydrogel by adding horseradish peroxidase (HRP) and hydrogen peroxide to a gelatin-hydroxyphenylacetic acid (GH) copolymer so that phenol derivatives in the GH copolymer cross-link with each other; and
mixing the injectable intraocular microgel in the GH hydrogel.

8. The method of claim 1, comprising:
mixing hyaluronic acid thiol (HA-SH) and hyaluronic acid vinylsulfone (HA-VS) so that a vinyl group of HA-VS and a thiol group of HA-SH cross-link with each other through a thiol-ene reaction, thereby obtaining a HA-VS/HA-SH hydrogel; and
mixing the injectable intraocular microgel in the HA-VS/HA-SH hydrogel.

9. An injectable intraocular hydrogel prepared by the methods of claim 1 comprising:
hyaluronic acid microgels distributed in the hydrogel; and a drug loaded on the hyaluronic acid microgels,
wherein the hyaluronic acid microgels composed of a hyaluronic acid copolymer cross-linked by a cross-linking functional represented by at least one Chemical Formula selected from the group consisting of Chemical Formulas 1-1 to 1-6 below.

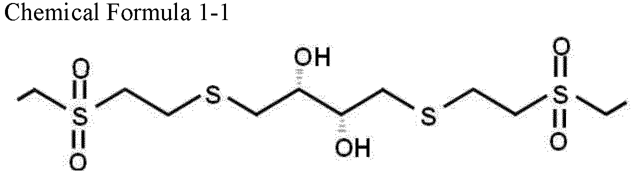

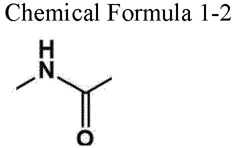

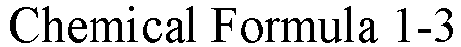

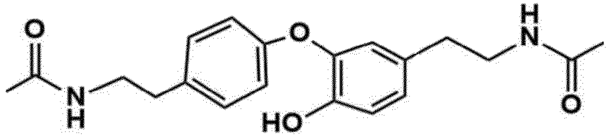

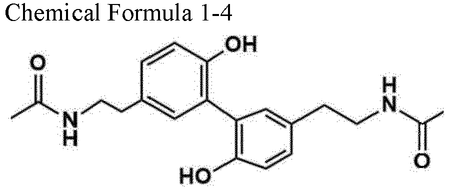

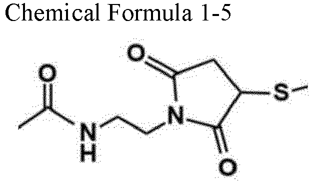

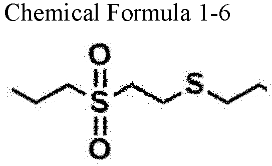

10. The injectable intraocular hydrogel of claim 9, wherein the hyaluronic acid copolymer is at least one selected from the group consisting of hyaluronic acid vinylsulfone (HA-VS), hyaluronic acid-(N-hydroxy succinimide) (HA-NHS), hyaluronic acid-tyramine (HA-TA), and hyaluronic acid-maleimide (HA-Mal).

11. The injectable intraocular hydrogel of claim 9, wherein the drug is one or more selected from an injectable intraocular protein therapeutic agent, a chemical drug, a small molecule therapeutics, a gene, and a cell.

12. The injectable intraocular hydrogel of claim 9, wherein the injectable intraocular microgels have an average size of 30 to 160 µm.

13. The injectable intraocular hydrogel of claim 9-12, wherein the hydrogel is cross-linked by a cross-linking functional group represented by at least one selected from the group consisting of Chemical Formulas 2-1 to 2-3 below:

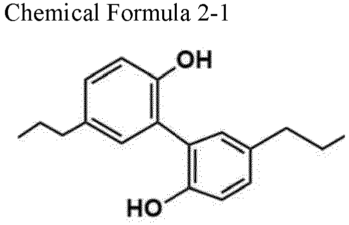

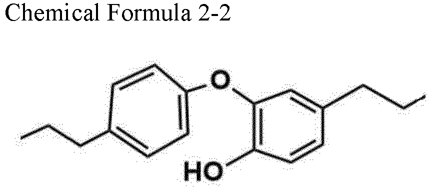

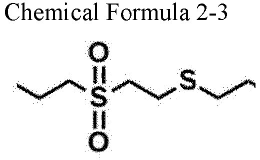

## Patentansprüche

1. Herstellverfahren eines injizierbaren intraokularen Hydrogels, wobei das Verfahren aufweist:
Erzeugen von Hyaluronsäure-Mikrogelen mittels Veranlassens eines Hyaluronsäure-Copolymers sich einer Vernetzungsreaktion eines W/O-Emulsionsverfahren zu unterziehen;
Laden eines Arzneimittels auf die Hyaluronsäure-Mikrogele; und
Verteilen der Hyaluronsäure-Mikrogelen in dem Hydrogel.

2. Verfahren nach Anspruch 1, wobei das Erzeugen der Hyaluronsäure-Mikrogele aufweist:
Synthetisieren eines Hyaluronsäure-Vinylsulfon (HA-VS)-Copolymers mittels Verursachen, dass sich Divinylsulfon (DVS) und Hyaluronsäure (HA) einer Bindungsreaktion unterziehen; und
Verursachen, dass sich das synthetisierte Hyaluronsäure-Vinylsulfon (HA-VS)-Copolymer und Dithiothreitol (DTT) einer Thiol-En-Vernetzungsreaktion unterziehen.

3. Verfahren nach Anspruch 1, wobei das Erzeugen der Hyaluronsäure-Mikrogele durch NHS-NH2-Vernetzen von Hyaluronsäure-(N-hydroxysuccinimid) (HA-NHS) und Hyaluronsäureamin (HA-NH₂) durchgeführt wird.

4. Verfahren nach Anspruch 1, wobei das Erzeugen der Hyaluronsäure-Mikrogele aufweist:
Zugeben von Meerrettichperoxidase (HRP) und Wasserstoffperoxid (H₂O₂) zu Hyaluronsäure-Tyramin (HA-TA); und
Veranlassen der resultierenden Mischung sich einer enzymatischen Vernetzung zu unterziehen.

5. Verfahren nach Anspruch 1, wobei das Erzeugen der Hyaluronsäure-Mikrogele durch Thiol-En-Vernetzen von Hyaluronsäure-Vinylsulfon (HA-VS) und Hyaluronsäurethiol (HA-SH) durchgeführt wird.

6. Verfahren nach Anspruch 1, welches aufweist:
Herstellen eines GPT-Hydrogels durch Zugeben von Meerrettichperoxidase (HRP) und Wasserstoffperoxid zu einem Gelatine-Polyethylenglykol-Tyramin (GPT)-Copolymer, sodass sich Phenolderivate im GPT-Copolymer miteinander vernetzen; und
Mischen des injizierbaren intraokularen Mikrogels in das GPT-Hydrogel.

7. Verfahren nach Anspruch 1, welches aufweist:
Herstellen eines GH-Hydrogels durch Zugeben von Meerrettichperoxidase (HRP) und Wasserstoffperoxid zu einem Gelatine-Hydroxyphenylessigsäure (GH)-Copolymer, sodass sich Phenolderivate im GH-Copolymer miteinander vernetzen; und
Mischen des injizierbaren intraokularen Mikrogels in das GH-Hydrogel.

8. Verfahren nach Anspruch 1, welches aufweist:
Mischen von Hyaluronsäurethiol (HA-SH) und Hyaluronsäure-Vinylsulfon (HA-VS), sodass eine Vinylgruppe von HA-VS und eine Thiolgruppe von HA-SH durch eine Thiol-En-Reaktion miteinander vernetzt werden, wodurch ein HA-VS/HA-SH-Hydrogel erhalten wird; und
Mischen des injizierbaren intraokularen Mikrogels in das HA-VS/HA-SH-Hydrogel.

9. Injizierbares intraokulares Hydrogel, welches nach den Verfahren nach Anspruch 1 hergestellt ist, welches aufweist:
Hyaluronsäure-Mikrogele, welche in dem Hydrogel verteilt sind; und ein Arzneimittel, welches auf die Hyaluronsäure-Mikrogele geladen ist,
wobei die Hyaluronsäure-Mikrogele aus einem Hyaluronsäure-Copolymer gebildet ist, das durch eine vernetzende funktionelle Gruppe vernetzt ist, welche durch zumindest eine chemische Formel dargestellt wird, die aus der Gruppe ausgewählt ist, die aus den untenstehenden chemischen Formeln 1-1 bis 1-6 besteht.

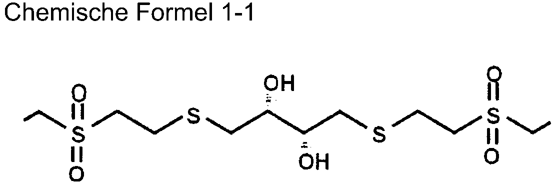

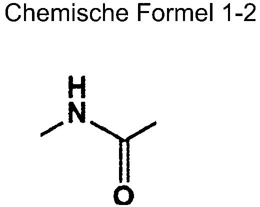

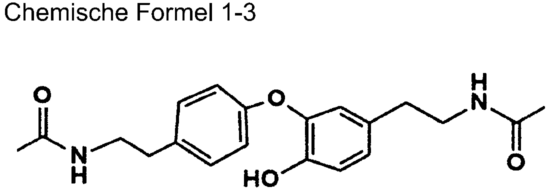

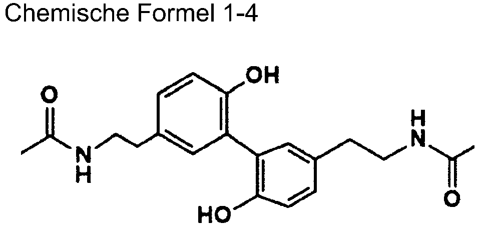

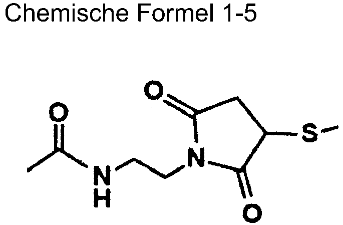

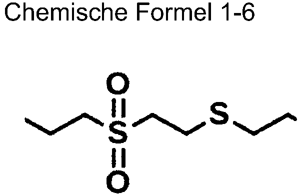

10. Injizierbares intraokulares Hydrogel nach Anspruch 9, wobei das Hyaluronsäure-Copolymer zumindest eines ist aus der Gruppe bestehend aus Hyaluronsäure-Vinylsulfon (HA-VS), Hyaluronsäure-(N-hydroxysuccinimid) (HA-NHS), Hyaluronsäure-Tyramin (HA-TA) und Hyaluronsäuremaleimid (HA-Mal) ausgewählt ist.

11. Injizierbares intraokulares Hydrogel nach Anspruch 9, wobei das Arzneimittel eines oder mehrere ausgewählt aus einem injizierbaren intraokularen Protein-Therapeutisches-Mittel, einem chemischen Arzneimittel, einem niedermolekularen Therapeutikum, einem Gen und einer Zelle ist.

12. Injizierbares intraokulares Hydrogel nach Anspruch 9, wobei die injizierbaren intraokularen Mikrogelen eine durchschnittliche Größe von 30 bis 160 µm haben.

13. Injizierbares intraokulares Hydrogel nach Anspruch 9-12, wobei das Hydrogel durch eine vernetzende funktionelle Gruppe vernetzt ist, die dargestellt ist durch zumindest eines ausgewählt aus der Gruppe bestehend aus den untenstehenden chemischen Formeln 2-1 bis 2-3:

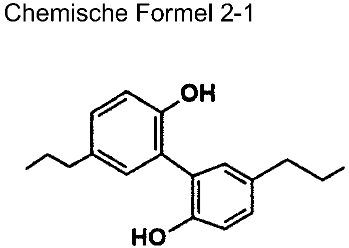

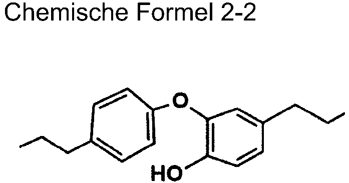

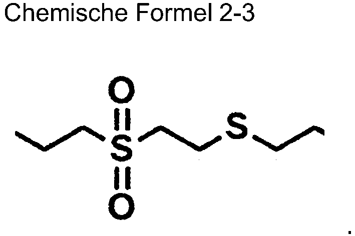

## Revendications

1. Procédé de préparation d'un hydrogel intraoculaire injectable, le procédé comprenant :
la génération de microgels d'acide hyaluronique en faisant subir à un copolymère d'acide hyaluronique une réaction de réticulation d'un procédé d'émulsion E/H ;
le chargement d'un médicament sur les microgels d'acide hyaluronique ; et
la distribution des microgels d'acide hyaluronique dans l'hydrogel.

2. Procédé selon la revendication 1, dans lequel la génération des microgels d'acide hyaluronique consiste à :
synthétiser un copolymère de vinylsulfone d'acide hyaluronique (HA-VS) en faisant subir une réaction de liaison à la divinylsulfone (DVS) et à l'acide hyaluronique (HA) ; et
faire subir au copolymère vinylsulfone d'acide hyaluronique (HA-VS) synthétisé et au dithiothréitol (DTT) une réaction de réticulation thiol-ène.

3. Procédé selon la revendication 1, dans lequel la génération des microgels d'acide hyaluronique est effectuée par réticulation NHS-NH2 d'acide hyaluronique-(N-hydroxy succinimide) (HA-NHS) et d'acide hyaluronique-amine (HA-NH₂).

4. Procédé selon la revendication 1, dans lequel la génération des microgels d'acide hyaluronique consiste à :
ajouter une peroxydase de raifort (HRP) et de peroxyde d'hydrogène (H₂O₂) à l'acide hyaluronique-tyramine (HA-TA) ; et
faire subir au mélange résultant une réticulation enzymatique.

5. Procédé selon la revendication 1, dans lequel la génération des microgels d'acide hyaluronique est effectuée par réticulation thiol-ène de vinylsulfone d'acide hyaluronique (HA-VS) et de thiol d'acide hyaluronique (HA-SH).

6. Procédé selon la revendication 1, comprenant :
la préparation d'un hydrogel de GPT en ajoutant de la peroxydase de raifort (HRP) et du peroxyde d'hydrogène à un copolymère de gélatine-polyéthylène glycol-tyramine (GPT) de sorte que les dérivés du phénol dans le copolymère GPT se réticulent les uns avec les autres ; et
le mélange du microgel intraoculaire injectable dans l'hydrogel GPT.

7. Procédé selon la revendication 1, comprenant :
la préparation d'un hydrogel de GH en ajoutant de la peroxydase de raifort (HRP) et du peroxyde d'hydrogène à un copolymère de gélatine et d'acide hydroxyphénylacétique (GH) de sorte que les dérivés du phénol dans le copolymère GH se réticulent les uns avec les autres ; et
le mélange du microgel intraoculaire injectable dans l'hydrogel GH.

8. Procédé selon la revendication 1, comprenant :
le mélange de thiol d'acide hyaluronique (HA-SH) et de vinylsulfone d'acide hyaluronique (HA-VS) de sorte qu'un groupe vinyle de HA-VS et un groupe thiol de HA-SH se réticulent par une réaction thiol-ène, obtenant ainsi un hydrogel HA-VS/HA-SH ; et
le mélange du microgel intraoculaire injectable dans l'hydrogel HA-VS/HA-SH.

9. Hydrogel intraoculaire injectable préparé par les procédés selon la revendication 1, comprenant :
des microgels d'acide hyaluronique distribués dans l'hydrogel ; et un médicament chargé sur les microgels d'acide hyaluronique,
dans lequel les microgels d'acide hyaluronique sont composés d'un copolymère d'acide hyaluronique réticulé par une fonction de réticulation représentée par au moins une formule chimique choisie dans le groupe constitué par les formules chimiques 1-1 à 1-6 ci-dessous.

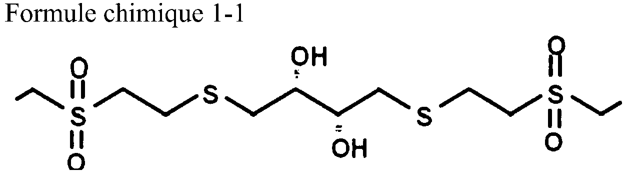

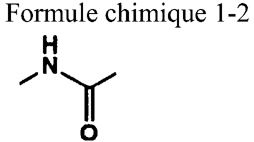

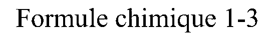

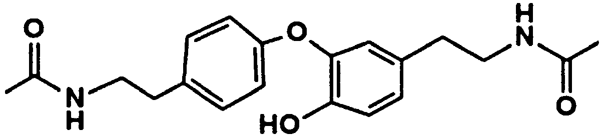

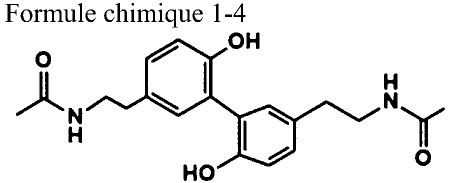

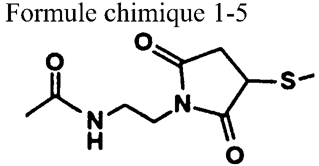

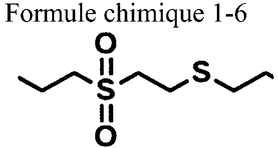

10. Hydrogel intraoculaire injectable selon la revendication 9, dans lequel le copolymère d'acide hyaluronique est au moins un copolymère choisi dans le groupe constitué par la vinylsulfone d'acide hyaluronique (HA-VS), l'acide hyaluronique- (N-hydroxy succinimide) (HA-NHS), l'acide hyaluronique-tyramine (HA-TA) et l'acide hyaluronique-maléimide (HA-Mal).

11. Hydrogel intraoculaire injectable selon la revendication 9, dans lequel le médicament est un ou plusieurs éléments choisis parmi un agent thérapeutique à base de protéine intraoculaire injectable, un médicament chimique, un agent thérapeutique à base de petites molécules, un gène et une cellule.

12. Hydrogel intraoculaire injectable selon la revendication 9, dans lequel les microgels intraoculaires injectables ont une taille moyenne de 30 à 160 µm.

13. Hydrogel intraoculaire injectable selon les revendications 9 à 12, dans lequel l'hydrogel est réticulé par un groupe fonctionnel de réticulation représenté par au moins un élément choisi dans le groupe constitué par les formules chimiques 2-1 à 2-3 ci-dessous :
